# EUROPEAN PATENT APPLICATION

(11) **EP 4 778 936 A1**
(43) Date of publication of application: **22.07.2026**
(21) Application number: 24913663.1
(22) Date of filing: 27.12.2024
(51) Int. Cl.: C07K 14/34, C12N 15/77

(54) **VARIANT OF TWIN-ARGININE TRANSLOCASE AND METHOD FOR PRODUCING TARGET PROTEIN USING SAME**

(30) Priority: 29.12.2023 KR 20230197062
(71) Applicant: CJ Cheiljedang Corporation, Seoul 04560 (KR)
(72) Inventor: HWANG, Seungho, Seoul 04560 (KR); PARK, Sung Eun, Seoul 04560 (KR); LEE, Heeseok, Seoul 04560 (KR)
(74) Representative: Meissner Bolte Partnerschaft mbB
(86) International application number: PCT/KR2024/021254
(87) International publication number: WO 2025/143858

(57) **Abstract**

The present application relates to a twin-arginine translocase subunit TatC variant, a microorganism comprising the TatC variant, a composition for producing a target protein, using the microorganism, and a method for producing the target protein. The microorganism comprising the TatC variant has the effect of improving the secretion and productivity of a target protein.

## Description

### [TECHNICAL FIELD]

### CROSS-REFERENCE TO RELATED PATENT APPLICATION(S)

The present disclosure claims the benefit of Korean Patent Application No. 10-2023-0197062, filed on December 29, 2023, in the Korean Intellectual Property Office, the disclosure of which is incorporated herein in its entirety by reference.

This application relates to a variant of a twin-arginine translocation protein and a method for producing a target protein using the same.

### [BACKGROUND ART]

A microorganism of the genus *Corynebacterium* has been used for a long time in the industrial field for the production of amino acids and nucleic acids, etc., and its safety has been recognized. The microorganism of the genus *Corynebacterium* possesses a translocation system for secreting proteins expressed within the cell to the outside of the cell, but it is known that the types of proteins inherently secreted outside the cell, including proteases, are very limited. Therefore, when the microorganism of the genus *Corynebacterium* is used to establish a system for extracellular secretion and production of a target protein, the recovery and purification of the target protein are facilitated, resulting in high industrial applicability.

Although there have also been studies to improve the secretion of the target protein among the prior literature that have attempted to utilize the microorganism of the genus *Corynebacterium* for the secretion and production of the target protein [Korean Patent Publication No. 10-2022-0049827], they have only been limited to enhancing expression levels of some genes of the protein translocation system inherently possessed by the microorganism of the genus *Corynebacterium*, and thus there is a clear limitation in an improvement effect of target protein secretion and production capability.

### [Prior Art Literature]

### [Patent Literature]

(Patent Literature 1) Republic of Korea Patent Publication No. KR 10-2022-0049827 A

### [DISCLOSURE]

### [TECHNICAL PROBLEM]

An object of the present disclosure is to provide a polypeptide comprising an amino acid sequence having 90% or more sequence homology to the amino acid sequence of SEQ ID NO: 1,
wherein (1) amino acids corresponding to the 106th and 107th residues from the N-terminus in the amino acid sequence of SEQ ID NO: 1 are substituted with different amino acids; or
(2) amino acids corresponding to the 106th, the 107th, and the 108th residues from the N-terminus in the amino acid sequence of SEQ ID NO: 1 are substituted with different amino acids.

Another object of the present disclosure is to provide a polynucleotide encoding the polypeptide.

Still another object of the present disclosure is to provide a recombinant vector comprising the polynucleotide.

Still another object of the present disclosure is to provide a microorganism for producing a target protein, comprising: the polypeptide and the target protein; or

a polynucleotide encoding the polypeptide and a gene encoding the target protein.

Still another object of the present disclosure is to provide a composition for producing a target protein, comprising the microorganism.

Yet another object of the present disclosure is to provide a method for producing a target protein, comprising culturing the microorganism in a medium.

### [TECHNICAL SOLUTION]

This will be described in detail as follows. Meanwhile, each of the descriptions and embodiments disclosed in the present disclosure may also be applied to each of other descriptions and embodiments. That is, all combinations of various elements disclosed in the present disclosure fall within the scope of the present disclosure. In addition, the scope of the present disclosure may not be considered limited by the specific description described below. In addition, those of ordinary skill in the art may recognize or confirm numerous equivalents to the specific aspects of the present disclosure described herein using only routine experiments. In addition, such equivalents are intended to be included in the present disclosure.

Hereinafter, the present disclosure will be described in more detail.

### Polypeptide

The present disclosure provides a polypeptide comprising:
an amino acid sequence having at least 90% or more sequence homology to the amino acid sequence of SEQ ID NO: 1; or the amino acid sequence of SEQ ID NO: 1,
wherein amino acid residues corresponding to the 106th and the 107th residues from the N-terminus in the amino acid sequence of SEQ ID NO: 1; or amino acid residues corresponding to the 106th, the 107th, and the 108th residues from the N-terminus in the amino acid sequence of SEQ ID NO: 1, are substituted with amino acids different from original amino acids.

The polypeptide may be a polypeptide having twin-arginine translocase activity.

The polypeptide may be a twin-arginine translocase subunit TatC protein.

In one embodiment, the twin-arginine translocase subunit TatC protein may be derived from *Corynebacterium glutamicum* (WP_011014400.1) and may be represented by the amino acid sequence of SEQ ID NO: 1.

In one embodiment, the polypeptide may comprise a sequence having 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more, or 99.9% or more sequence homology to the amino acid sequence of SEQ ID NO: 1,
wherein (1) amino acids corresponding to the 106th and the 107th residues from the N-terminus in the amino acid sequence of SEQ ID NO: 1 are substituted with different amino acids; or
(2) amino acids corresponding to the 106th, the 107th, and the 108th residues from the N-terminus in the amino acid sequence of SEQ ID NO: 1 are substituted with different amino acids.

In one embodiment, the polypeptide may be a polypeptide comprising the amino acid sequence of SEQ ID NO: 1,
wherein (1) the 106th and the 107th amino acids from the N-terminus in the amino acid sequence of SEQ ID NO: 1 are substituted with different amino acids; or
(2) the 106th, the 107th, and the 108th amino acids from the N-terminus in the amino acid sequence of SEQ ID NO: 1 are substituted with different amino acids.

The amino acid corresponding to the 106th residue from the N-terminus in the amino acid sequence of SEQ ID NO: 1 or the 106th amino acid from the N-terminus in the amino acid sequence of SEQ ID NO: 1 may be threonine.

Accordingly, the amino acid corresponding to the 106th residue or the 106th amino acid may be substituted with alanine, arginine, histidine, lysine, aspartic acid, glutamic acid, serine, asparagine, glutamine, cysteine, glycine, proline, valine, isoleucine, leucine, methionine, phenylalanine, tyrosine, or tryptophan. In one specific embodiment, the amino acid corresponding to the 106th residue or the 106th amino acid may be substituted with alanine.

The amino acid corresponding to the 107th residue from the N-terminus in the amino acid sequence of SEQ ID NO: 1 or the 107th amino acid from the N-terminus in the amino acid sequence of SEQ ID NO: 1 may be proline.

Accordingly, the amino acid corresponding to the 107th residue or the 107th amino acid may be substituted with serine, arginine, histidine, lysine, aspartic acid, glutamic acid, threonine, asparagine, glutamine, cysteine, glycine, alanine, valine, isoleucine, leucine, methionine, phenylalanine, tyrosine, or tryptophan. In one specific embodiment, the amino acid corresponding to the 107th residue or the 107th amino acid may be substituted with serine.

The amino acid corresponding to the 108th residue from the N-terminus in the amino acid sequence of SEQ ID NO: 1 or the 108th amino acid from the N-terminus in the amino acid sequence of SEQ ID NO: 1 may be glycine.

Accordingly, the amino acid corresponding to the 108th residue or the 108th amino acid may be substituted with alanine, arginine, histidine, lysine, aspartic acid, glutamic acid, serine, threonine, asparagine, glutamine, cysteine, proline, valine, isoleucine, leucine, methionine, phenylalanine, tyrosine, or tryptophan. In one specific embodiment, the amino acid corresponding to the 108th residue or the 108th amino acid may be substituted with alanine.

In one embodiment, the polypeptide may be a polypeptide comprising
a sequence having at least 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more, or 99.9% or more sequence homology to the amino acid sequence of SEQ ID NO: 1,
wherein (1) the amino acid corresponding to the 106th residue from the N-terminus in the amino acid sequence of SEQ ID NO: 1 is substituted with alanine, arginine, histidine, lysine, aspartic acid, glutamic acid, serine, asparagine, glutamine, cysteine, glycine, proline, valine, isoleucine, leucine, methionine, phenylalanine, tyrosine, or tryptophan, and the amino acid corresponding to the 107th residue is substituted with serine, arginine, histidine, lysine, aspartic acid, glutamic acid, threonine, asparagine, glutamine, cysteine, glycine, alanine, valine, isoleucine, leucine, methionine, phenylalanine, tyrosine, or tryptophan; or
(2) the amino acid corresponding to the 106th residue from the N-terminus in the amino acid sequence of SEQ ID NO: 1 is substituted with alanine, arginine, histidine, lysine, aspartic acid, glutamic acid, serine, asparagine, glutamine, cysteine, glycine, proline, valine, isoleucine, leucine, methionine, phenylalanine, tyrosine, or tryptophan, the amino acid corresponding to the 107th residue is substituted with serine, arginine, histidine, lysine, aspartic acid, glutamic acid, threonine, asparagine, glutamine, cysteine, glycine, alanine, valine, isoleucine, leucine, methionine, phenylalanine, tyrosine, or tryptophan, and the amino acid corresponding to the 108th residue is substituted with alanine, arginine, histidine, lysine, aspartic acid, glutamic acid, serine, threonine, asparagine, glutamine, cysteine, proline, valine, isoleucine, leucine, methionine, phenylalanine, tyrosine, or tryptophan.

In one embodiment, the polypeptide may be a polypeptide comprising
a sequence having at least 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more, or 99.9% or more sequence homology to the amino acid sequence of SEQ ID NO: 1,
wherein (1) the amino acid corresponding to the 106th residue from the N-terminus in the amino acid sequence of SEQ ID NO: 1 is substituted with alanine and the amino acid corresponding to the 107th residue is substituted with serine; or
(2) the amino acid corresponding to the 106th residue from the N-terminus in the amino acid sequence of SEQ ID NO: 1 is substituted with alanine, the amino acid corresponding to the 107th residue is substituted with serine, and the amino acid corresponding to the 108th residue is substituted with alanine.

In one embodiment, the polypeptide may be a polypeptide in which:
(1) the 106th amino acid in the amino acid sequence of SEQ ID NO: 1 is substituted with alanine, arginine, histidine, lysine, aspartic acid, glutamic acid, serine, asparagine, glutamine, cysteine, glycine, proline, valine, isoleucine, leucine, methionine, phenylalanine, tyrosine, or tryptophan, and the 107th amino acid is substituted with serine, arginine, histidine, lysine, aspartic acid, glutamic acid, threonine, asparagine, glutamine, cysteine, glycine, alanine, valine, isoleucine, leucine, methionine, phenylalanine, tyrosine, or tryptophan; or
(2) the 106th amino acid from the N-terminus in the amino acid sequence of SEQ ID NO: 1 is substituted with alanine, arginine, histidine, lysine, aspartic acid, glutamic acid, serine, asparagine, glutamine, cysteine, glycine, proline, valine, isoleucine, leucine, methionine, phenylalanine, tyrosine, or tryptophan, the 107th amino acid is substituted with serine, arginine, histidine, lysine, aspartic acid, glutamic acid, threonine, asparagine, glutamine, cysteine, glycine, alanine, valine, isoleucine, leucine, methionine, phenylalanine, tyrosine, or tryptophan, and the 108th amino acid is substituted with alanine, arginine, histidine, lysine, aspartic acid, glutamic acid, serine, threonine, asparagine, glutamine, cysteine, proline, valine, isoleucine, leucine, methionine, phenylalanine, tyrosine, or tryptophan.

In one embodiment, the polypeptide may be a polypeptide in which:
(1) the 106th amino acid from the N-terminus in the amino acid sequence of SEQ ID NO: 1 is substituted with alanine and the 107th amino acid is substituted with serine; or
(2) the 106th amino acid from the N-terminus in the amino acid sequence of SEQ ID NO: 1 is substituted with alanine, the 107th amino acid is substituted with serine, and the 108th amino acid is substituted with alanine.

In one specific embodiment, the polypeptide may be a polypeptide comprising the amino acid sequence of SEQ ID NO: 1,
wherein (1) the 106th amino acid from the N-terminus in the amino acid sequence of SEQ ID NO: 1 is substituted with alanine and the 107th amino acid is substituted with serine; or
(2) the 106th amino acid from the N-terminus in the amino acid sequence of SEQ ID NO: 1 is substituted with alanine, the 107th amino acid is substituted with serine, and the 108th amino acid is substituted with alanine.

In one specific embodiment, the polypeptide may comprise the amino acid sequence of SEQ ID NO: 18 or SEQ ID NO: 24, or may consist of said amino acid sequence.

When the polypeptide comprises a mutation in which the amino acid residues corresponding to the 106th and the 107th residues from the N-terminus in the amino acid sequence of SEQ ID NO: 1; or the amino acid residues corresponding to the 106th, the 107th, and the 108th residues are substituted with amino acids different from original amino acids, it is obvious that the polypeptide may be included in the polypeptide of the present disclosure as long as it exhibits twin-arginine translocase activity, even when amino acid residues other than the amino acid residues are deleted, modified, substituted, or added. For example, it may be a case of having addition or deletion of a sequence that does not alter activity of a polypeptide at the N-terminus, the C-terminus, and/or inside of the amino acid sequence of the polypeptide, a naturally occurring mutation, a silent mutation, or a conservative substitution.

The "conservative substitution" refers to substituting one amino acid with another amino acid having similar structural and/or chemical properties. Such an amino acid substitution may generally occur based on similarities in polarity, charge, solubility, hydrophobicity, hydrophilicity, and/or amphipathic nature of residues. Conventionally, the conservative substitution may have almost no effect or no effect on activity of a protein or a polypeptide.

In one embodiment, the polypeptide may be a polypeptide comprising a sequence having at least 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more, or 99.9% or more sequence homology to the amino acid sequence of SEQ ID NO: 1,
wherein (1) the amino acid corresponding to the 106th and the 107th residues from the N-terminus in the amino acid sequence of SEQ ID NO: 1 are substituted with different amino acids; or
(2) the amino acids corresponding to the 106th, the 107th, and the 108th residues from the N-terminus in the amino acid sequence of SEQ ID NO: 1 are substituted with different amino acids,
and having twin-arginine translocase activity.

In one embodiment, the polypeptide having twin-arginine translocase activity may be a twin-arginine translocase subunit TatC protein.

In one embodiment, the polypeptide (e.g., polypeptide having twin-arginine translocase activity) or the twin-arginine translocase subunit TatC protein may be derived from a microorganism of the genus *Corynebacterium*. The microorganism of the genus *Corynebacterium* may be one or more microorganisms selected from the group consisting of *Corynebacterium glutamicum*, *Corynebacterium crudilactis*, *Corynebacterium deserti*, *Corynebacterium efficiens*, *Corynebacterium callunae*, *Corynebacterium stationis*, *Corynebacterium singulare*, *Corynebacterium halotolerans*, *Corynebacterium striatum*, *Corynebacterium pollutisoli*, *Corynebacterium imitans*, *Corynebacterium testudinoris*, and *Corynebacterium flavescens,* but is not limited thereto.

In the present disclosure, the term "variant polypeptide" refers to a polypeptide in which one or more amino acids are conservatively substituted and/or modified, so that the variant polypeptide is different from the amino acid sequence before mutation, but the functions or properties are maintained. Such a variant polypeptide may generally be identified by modifying one or more amino acids in the amino acid sequence of the polypeptide and evaluating the properties of the modified polypeptide. That is, the ability of the variant polypeptide may be increased, remain unchanged, or reduced compared to the polypeptide before mutation. In addition, some variant polypeptides may include a variant polypeptide in which one or more parts, such as an N-terminus leader sequence or a transmembrane domain, are removed. Other variant polypeptides may include a variant polypeptide in which a part is removed from the N-terminus and/or the C-terminus of a mature protein. The term "variant polypeptide" may be interchangeably used with terms such as variant, modified polypeptide, mutated protein, mutant, and mutein, and the like (as English expressions, modification, modified polypeptide, modified protein, mutant, mutein, divergent, variant, etc.), and it is not limited thereto, as long as it is a term used as a meaning of being mutated. In addition, the variant polypeptide may comprise deletion or addition of amino acids having minimal effects on the properties and secondary structure of the polypeptide. For example, at the N-terminus of the variant polypeptide, a signal (or leader) sequence involved in protein translocation co-translationally or post-translationally may be conjugated. In addition, the variant polypeptide may be conjugated with another sequence or linker to enable identification, purification, or synthesis.

For the purpose of the present disclosure, the variant polypeptide may be a polypeptide comprising a sequence having 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more, or 99.9% or more sequence homology to the amino acid sequence of SEQ ID NO: 1,
wherein (1) the amino acids corresponding to the 106th and the 107th residues from the N-terminus in the amino acid sequence of SEQ ID NO: 1 are substituted with different amino acids; or
(2) the amino acids corresponding to the 106th, the 107th, and the 108th residues from the N-terminus in the amino acid sequence of SEQ ID NO: 1 are substituted with different amino acids.

The variant polypeptide may have increased twin-arginine translocase activity and/or increased activity of increasing secretion and production capability of a microorganism for a target protein, compared to the polypeptide before mutation (e.g., a polypeptide comprising the amino acid sequence of SEQ ID NO: 1).

### Polynucleotide

Another aspect provides a polynucleotide encoding the polypeptide.

In the present disclosure, the term "polynucleotide" may refer to a DNA or RNA strand of a certain length or more, which is a polymer of nucleotides in which nucleotide monomers are linked in a long chain shape by covalent bonds.

The polynucleotide may comprise a sequence having 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more, or 99.9% or more sequence homology to the nucleic acid sequence of SEQ ID NO: 2,
wherein codons encoding the amino acid residues corresponding to the 106th and the 107th residues from the N-terminus in the amino acid sequence of SEQ ID NO: 1 are substituted with codons encoding different amino acids, or
codons encoding the amino acid residues corresponding to the 106th, the 107th, and the 108th residues from the N-terminus in the amino acid sequence of SEQ ID NO: 1 are substituted with codons encoding different amino acids.

It was described above that the amino acid corresponding to the 106th residue from the N-terminus in the amino acid sequence of SEQ ID NO: 1 or the 106th amino acid from the N-terminus in the amino acid sequence of SEQ ID NO: 1 is threonine, the amino acid corresponding to the 107th residue or the 107th amino acid is proline, and the amino acid corresponding to the 108th residue or the 108th amino acid is glycine.

Accordingly, codons encoding the amino acid corresponding to the 106th residue from the N-terminus in the amino acid sequence of SEQ ID NO: 1 or the 106th amino acid from the N-terminus in the amino acid sequence of SEQ ID NO: 1 may be ACU, ACC, ACA or ACG, codons encoding the amino acid corresponding to the 107th residue or the 107th amino acid may be CCU, CCC, CCA or CCG, and codons encoding the amino acid corresponding to the 108th residue or the 108th amino acid may be GGU, GGC, GGA or GGG.

It is well known in the art which polynucleotide sequences are comprised in codons encoding each amino acid. The codons may be modified in various ways within a range that does not change the amino acid sequence of the polypeptide by considering degeneracy of codons or codons preferred in an organism in which the polypeptide is to be expressed.

In one specific embodiment, the polynucleotide may comprise the nucleic acid sequence of SEQ ID NO: 19 or SEQ ID NO: 25, or may consist of the nucleic acid sequence of SEQ ID NO: 19 or SEQ ID NO: 25.

The polynucleotide of the present disclosure may include, without limitation, a probe that may be prepared from a known gene sequence, for example, a sequence which may hybridize under a stringent condition with a complementary sequence to all or a part of the polynucleotide sequence of the present disclosure. The term "stringent condition" refers to a condition that allows specific hybridization between polynucleotides. Such a condition is specifically described in the literature (see J. Sambrook et al.,Molecular Cloning, A Laboratory Manual, 2nd Edition, Cold Spring Harbor Laboratory press, Cold Spring Harbor, New York, 1989; F.M. Ausubel et al.,Current Protocols in Molecular Biology, John Wiley & Sons, Inc., New York, 9.50-9.51, 11.7-11.8). For example, the following may be listed: conditions under which polynucleotides having high homology or identity hybridize with each other, or polynucleotides having at least 80% or more, 81% or more, 82% or more, 83% or more, 84% or more, 85% or more, 86% or more, 87% or more, 88% or more, 89% or more, 90% or more, 90.5% or more, 91% or more, 91.5% or more, 92% or more, 92.5% or more, 93% or more, 93.5% or more, 94% or more, 94.5% or more, 95% or more, 95.5% or more, 96% or more, 96.5% or more, 97% or more, 97.5% or more, 98% or more, 98.5% or more, 99% or more, 99.5% or more, or 99.9% or more homology or identity hybridize with each other, and polynucleotides having homology or identity lower than the foregoing do not hybridize with each other, or washing conditions of a conventional southern hybridization, i.e., conditions of washing once, specifically two to three times, at a salt concentration and temperature corresponding to 60 °C, 1×SSC, 0.1% SDS, specifically 60 °C, 0.1×SSC, 0.1% SDS, and more specifically 68 °C, 0.1×SSC, 0.1% SDS.

Hybridization requires two nucleotides to be complementary sequences, but hybridized polynucleotides may comprise some mismatches between bases depending on the stringency of hybridization. The term "complementary" is used to describe a relationship between nucleotide bases that may be hybridized with each other. For example, with respect to DNA, adenine is complementary to thymine and cytosine is complementary to guanine. Accordingly, the polynucleotide of the present disclosure may also comprise not only a substantially similar nucleic acid sequence, but also an isolated nucleic acid fragment complementary to the entire sequence.

Specifically, a polynucleotide having homology or identity to the polynucleotide of the present disclosure may be detected using a hybridization condition comprising a hybridization step at a Tm value of 55 °C and using the conditions described above. In addition, the Tm value may be 60 °C, 63 °C, or 65 °C, but is not limited thereto, and may be appropriately controlled by those skilled in the art depending on the purpose.

Appropriate stringency for hybridizing the polynucleotide depends on the length of the polynucleotide and the degree of complementarity, and the variables are well known in the art (e.g., J. Sambrook et al., supra).

In the present disclosure, "consisting of a sequence," "essentially consisting of a sequence," or "comprising a sequence" may refer to all cases of comprising the sequence, but are not intended to exclude cases of comprising sequences other than the sequence.

In the present disclosure, the phrase "a polynucleotide (which may be interchangeably used with "gene") or a polypeptide (which may be interchangeably used with "protein") comprises, or consists of, or is represented by a specific nucleic acid sequence or amino acid sequence" may mean that the polynucleotide or polypeptide essentially comprises the specific nucleic acid sequence or amino acid sequence, and may be interpreted to comprise a "substantially equivalent sequence" in which insignificant mutations (deletion, substitution, modification, and/or addition) are applied to the specific nucleic acid sequence or amino acid sequence within a range of maintaining the original function and/or desired function of the polynucleotide or polypeptide (or may be interpreted not to exclude the insignificant mutations).

In the present disclosure, the term "homology" or "identity" refers to a degree of similarity between two given amino acid sequences or nucleotide sequences and may be expressed as a percentage. The terms homology and identity may often be used interchangeably.

Sequence homology or identity of a conserved polynucleotide or polypeptide may be determined by a standard alignment algorithm, and a default gap penalty established by a program being used may be used together. Substantially, homologous or identical sequences may generally hybridize with all or part of the sequence under moderate or highly stringent conditions. It is obvious that hybridization also comprises hybridization with a polynucleotide containing general codons or codons considering codon degeneracy in a polynucleotide.

Whether any two polynucleotide or polypeptide sequences have homology, similarity, or identity may be determined using known computer algorithms such as the "FASTA" program using default parameters, for example, as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453), as performed in Needleman program of EMBOSS package (EMBOSS: The European Molecular Biology Open Software Suite, Rice et al., 2000, Trends Genet. 16: 276-277) (version 5.0.0 or later version)(comprises GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, BLAST from the National Center for Biotechnology Information Database, or ClustalW may be used to determine homology, similarity, or identity.

Homology, similarity, or identity of a polynucleotide or a polypeptide may be determined by comparing sequence information using a GAP computer program such as Needleman et al. (1970), J Mol Biol. 48:443, for example, as known in Smith and Waterman, Adv. Appl. Math (1981) 2:482. In summary, the GAP program may be defined as a value of dividing a number of similar aligned symbols (i.e., nucleotide or amino acid) by a total number of symbols in the shorter one of the two sequences. Default parameters for the GAP program may comprise (1) a binary comparison matrix (containing values of 1 for identity and 0 for non-identity) and a weighted comparison matrix of Gribskov et al(1986) Nucl. Acids Res. 14: 6745, as disclosed by Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional penalty of 0.10 for each symbol in each gap (or a gap opening penalty of 10, a gap extension penalty of 0.5); and (3) no penalty for a terminal gap.

In the present disclosure, the term "corresponding to" refers to an amino acid residue at a position recited in a polypeptide, or an amino acid residue that is similar to, identical to, or homologous to the residue recited in the polypeptide. Identifying an amino acid at a corresponding position may be determining a specific amino acid in a sequence with reference to a specific sequence. The term "corresponding region" used in the present disclosure generally refers to a similar or corresponding position in a related protein or a reference protein.

For example, any amino acid sequence may be aligned with SEQ ID NO: 1, and based on this, each amino acid residue of the amino acid sequence may be numbered by referring to a numerical position of the amino acid residue corresponding to the amino acid residue of SEQ ID NO: 1. For example, a sequence alignment algorithm described in the present disclosure may identify a position of an amino acid, or a position at which a modification such as substitution, insertion, or deletion occurs, by comparison with a query sequence (also referred to as a "reference sequence").

In such alignment, for example, Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277), etc. may be used, but is not limited thereto, and any sequence alignment program, pairwise sequence comparison algorithm, etc. known in the art may be appropriately used.

### Recombinant vector

Still another aspect provides a recombinant vector comprising the polynucleotide.

In the present disclosure, the term "vector" refers to a DNA construct for delivering a target polynucleotide into an appropriate host or host cell. As an example, it may comprise a nucleic acid sequence of a polynucleotide encoding a target polypeptide which is operably linked to an appropriate expression regulatory region (or expression regulatory sequence) to enable expression of the target polypeptide within the appropriate host cell, but is not limited thereto. The expression regulatory region may comprise a promoter capable of initiating transcription, any operator sequence for regulating transcription, a sequence encoding an appropriate mRNA ribosome binding site, and/or a sequence regulating termination of transcription and/or translation. The vector may be transformed into an appropriate host cell, then maintained independently of the genome of the host cell, or inserted into the genome of the host cell. As an example, a target polynucleotide may be inserted into a chromosome through an insertion vector. The insertion of the polynucleotide into a chromosome may be accomplished by any method known in the art, for example, by homologous recombination, but is not limited thereto.

The vector that may be used in the present disclosure is not particularly limited as long as it may be replicated within a host cell, and may be selected from all conventionally used vectors. Examples of the conventionally used vectors may include plasmids, cosmids, viruses, bacteriophages, etc. in a natural state or recombined state. For example, as the vector, pWE15, M13, MBL3, MBL4, IXII, ASHII, APII, t10, t11, Charon4A, and Charon21A, etc. may be used as a phage vector or cosmid vector, and pBR series, pUC series, pBluescriptII series, pGEM series, pTZ series, pCL series, and pET series, etc. may be used as a plasmid vector. Specifically, pDZ, pACYC177, pACYC184, pCL, pECCG117, pUC19, pBR322, pMW118, pCC1BAC vectors, etc. may be exemplified, but are not limited thereto.

The vector may further comprise a selection marker for confirming whether it is introduced into a transformed cell or whether it is inserted into the genome of the transformed cell. The selection marker is for confirming whether a cell is transformed with the vector or whether the polynucleotide is inserted, and may be selected from genes that confer selectable phenotypes such as drug resistance, nutritional requirement, resistance to a cytotoxic agent, or expression of a surface protein. In an environment treated with a selective agent, only cells expressing the selection marker survive or exhibit different phenotypes, and thus transformed cells may be selected.

Expression of the polypeptide in a microorganism may be performed by introducing a polynucleotide encoding the polypeptide or a vector comprising the polynucleotide into a host cell and culturing a recombinant cell (e.g., microorganism) comprising the same.

Introduction of the polynucleotide encoding the polypeptide or a vector comprising the polynucleotide into a microorganism may be performed by those skilled in the art by appropriately selecting a known transformation method. In the present disclosure, the term "transformation" refers to changing genetic characteristics of the host cell (microorganism) by introducing the vector comprising a target polynucleotide or the polynucleotide into the host cell (microorganism). The transformed polynucleotide may be inserted into and positioned within a chromosome of a host cell or may be positioned outside the chromosome. The polynucleotide may be introduced in an appropriate form depending on a purpose of introduction. For example, the polynucleotide may be introduced into a host cell in a form of an expression cassette, which is a gene construct that comprises all elements required for self-expression. The expression cassette may generally comprise expression regulatory elements such as a promoter operably linked to the polynucleotide, a transcription termination signal, a ribosome binding site, and/or a translation termination signal. The expression cassette may be in a form of an expression vector capable of self-replication. In addition, the polynucleotide may be introduced into a host cell in its native form, and be operably linked to a sequence required for expression in the host cell. The term "operably linked" above may refer to that an expression regulatory element (e.g., a promoter) and the polynucleotide are functionally linked so that transcription regulation (e.g., transcription initiation) of the polynucleotide may be performed. The operable linkage may be performed using a gene recombination technique known in the art.

A method for transforming the polynucleotide into a host cell may be performed by any method for introducing nucleic acids into a cell (microorganism), and may be performed by appropriately selecting a transformation technique known in the art depending on the host cell. As the known transformation method, electroporation, calcium phosphate (CaPO₄) precipitation, calcium chloride (CaCl₂) precipitation, microinjection, polyethylene glycol (PEG) precipitation (polyethylene glycol-mediated uptake), DEAE-dextran method, cationic liposome method, lipofection, lithium acetate-DMSO method, etc. may be exemplified, but is not limited thereto.

The recombinant vector may comprise the polynucleotide and a gene encoding a target protein.

The target protein may refer to all types of protein having biologically useful activity that are intended to be produced using protein expression, secretion, and production system of a microorganism, and may be a protein desired to be produced in large quantities by those skilled in the art, and may refer to all proteins that may be expressed in a host cell by inserting a polynucleotide encoding the protein into a recombinant vector. For example, the target protein may be collagen, polypeptide derived from collagen, hormone, hormone analog, cytokine, antigen, an antigen binding fragment, antibody, cell receptor, enzyme, translocation protein, structural protein, serum, cell protein, antibiotic peptide, antioxidant peptide, etc.

In one embodiment, the target protein may be one or more selected from the group consisting of collagen (e.g., collagen derived from human, pig, cow, chicken, fish, etc.), polypeptide derived from collagen (polypeptide comprising amino acid sequence derived from collagen), etanercept, epoetin alpha, infliximab, interferon beta, insulin lispro, filgrastim, imiglucerase, glatiramer acetate, rituximab, pegfilgrastim, insulin grastim, adalimumab, trastuzumab, bevacizumab, ranibizumab, insulin, growth hormone, tumor necrosis factor-alpha, interleukin-7, insulin-like growth factor 2, interferon gamma, interferon alpha, interleukin-2, osteogenic protein, recombinant plasminogen-activator, bone morphogenetic protein 2, antifungal peptide, tissue plasminogen activator, immunoglobulin G, erythropoietin, granulocyte-macrophage stimulating factor receptor, granulocyte-colony stimulating factor, muromomab, abciximab, daclizumab, basiliximab, palivizumab, ibritumomab, omalizumab, efalizumab, tositumomab, cetuximab, natalizumab, alkaline phosphatase (PhoA), levan fructotransferase (LFT), cellulose binding domain, cholera toxin B, organophosphohydrolase, calcitonin, blood coagulation factors, hirudin, and monoclonal antibody 5T4, but is not limited thereto. In one embodiment, the target protein may further comprise a tag for protein purification, improvement of expression, dissolution, or detection of the target protein. Various tags usable for such purpose are known in the art and may be, for example, GST tag, FLAG tag, polyarginine tag, polyhistidine tag, for example, 6His-tag, MBP tag, S-tag, influenza virus HA tag, thioredoxin tag, or staphylococcus aureus protein A tag, etc.

In one embodiment, the target protein may be a collagen protein derived from bovine (bovine COL6A1, NP_001137337.1; SEQ ID NO: 5) or a polypeptide derived therefrom (SEQ ID NO: 6 or SEQ ID NO: 32), or a YGK peptide (SEQ ID NO: 34) which is an antioxidant-functional protein derived from *Saccharomyces cerevisiae*, but is not limited thereto.

In one embodiment, the target protein may be a protein and/or peptide having a desired activity in vivo (e.g., activity for preventing, alleviating, and/or treating a specific disease or symptom, or activity for replacing a biologically required substance), and may be, for example, one or more selected from the group consisting of a protein or peptide having enzymatic activity (e.g., protease, kinase, phosphatase, etc.), receptor protein or peptide, translocation protein or peptide, bactericidal and/or endotoxin-binding polypeptide, structural protein or peptide, immune polypeptide, toxin, antibiotic agent, hormone, growth factor, or vaccine, etc. In one embodiment, the target polypeptide may be one or more selected from the group consisting of a hormone, cytokine, tissue plasminogen activator, and immunoglobulin, (e.g., an antibody or an antigen binding fragment thereof or variant), etc. The immunoglobulin may be of any isotype (e.g., IgA, IgD, IgG, IgM, or IgE) and may be, for example, an IgG (e.g., IgG1, IgG2, IgG3, or IgG4) molecule. The antigen binding fragment may be a fragment that possesses an antigen binding ability of the original antibody, and may be any fragment of the antibody that comprises about 20 or more amino acids, for example, about 100 or more amino acids. The antigen binding fragment may be one or more selected from the group consisting of an antigen binding site of an antibody, such as CDS, Fab fragment, F(ab)2 fragment, Fv, scFv, a multibody comprising multiple binding domains (e.g., a diabody, a triabody, a tetrabody, etc.), a single domain antibody, or an affibody, etc. The antibody variant is a derivative of an antibody or antibody fragment having the same binding function as the antibody, but having an altered amino acid sequence from the original antibody. The antibody and/or antigen binding fragment may be, for example, mouse antibody, human antibody, chimeric antibody, humanized antibody, or human antibody. The antibody and/or antigen binding fragment may be isolated from a living body, or may be non-naturally occurring. The antibody and/or antigen binding fragment may be produced synthetically or recombinantly. The antibody may be a monoclonal antibody. In another specific embodiment, the target polypeptide may be one or more selected from the group consisting of various growth factors such as insulin, human growth hormone (hGH), insulin-like growth factor, EGF, VERF, various receptors, tissue plasminogen activator (tPA), erythropoietin (EPO), cytokine (e.g., interleukins such as IL-1, IL-2, IL-3, IL-4, IL-5, IL-6, IL-7, IL-8, IL-9, IL-10, IL-11, IL-12, IL-13, IL-14, IL-15, IL-16, IL-17, IL-18), interferon (IFN)-alpha, -beta, -gamma, -omega, or -tau, tumor necrosis factor (TNF) such as TNF-alpha, beta, or gamma, TRAIL, G-CSF, GM-CSF, M-CSF, or MCP-1, etc.

The recombinant vector may further comprise a gene encoding a secretion signal peptide. For expression and secretion of a target protein through a translocation system of a microorganism, the target protein may be linked to the secretion signal peptide at the N'-terminus. The recombinant vector may comprise the gene encoding the target protein and the gene encoding the secretion signal peptide in an appropriate order so as to express a form in which the secretion signal peptide is linked to the N'-terminus of the target protein.

The secretion signal peptide that may induce secretion of a target protein in a microorganism may be used without limitation, and in one embodiment, the secretion signal peptide may be one or more selected from the group consisting of Cg0955 secretion signal peptide, CgR0079 secretion signal peptide, CgR0120 secretion signal peptide, CgR0124 secretion signal peptide, CgR0900 secretion signal peptide, CgR0949 secretion signal peptide, CgR1023 secretion signal peptide, CgR1448 secretion signal peptide, CgR2137 secretion signal peptide, CgR2627 secretion signal peptide, CgR2926 secretion signal peptide, and TorA secretion signal peptide derived from *Escherichia coli* (*E*. *coli*), and IMD secretion signal peptide derived from *Arthrobacter globiformis*, but is not limited thereto. That is, any secretion signal peptide capable of inducing secretion of the target protein in a microorganism may be used without limitation.

The recombinant vector may comprise a promoter operably linked to the gene encoding the target protein and/or the gene encoding the secretion signal peptide for expression of the gene encoding the target protein and/or the gene encoding the secretion signal peptide.

In the present disclosure, the term "promoter" may refer to a non-translated nucleotide sequence upstream or downstream of an encoding region comprising a binding site for polymerase and having transcription initiation activity of a promoter target gene (e.g., a gene encoding a target protein or a twin-arginine translocase, etc.), for example, a DNA region to which the polymerase binds to initiate transcription of the gene.

Any promoter sequence generally used for expression of a gene may be used as the promoter, and examples of known promoters may be CJ1 to CJ7 promoters (US Patent No. US 7662943 B2), lac promoter, trp promoter, trc promoter, tac promoter, lambda phage PR promoter, PL promoter, tet promoter, gapA promoter, SPL7 promoter, SPL13(sm3) promoter (US Patent No. US 10584338 B2), O2 promoter (US Patent No. US 10273491 B2), tkt promoter, yccA promoter, etc., but are not limited thereto.

In one embodiment, any promoter capable of regulating transcription initiation in a cell, for example, a viral cell, a bacterial cell, a eukaryotic cell, an insect cell, a plant cell, or an animal cell, may be used as the promoter without limitation. For example, the promoter may be one or more selected from the group consisting of a promoter of prokaryotic cells or mammalian virus such as a cytomegalovirus (CMV) promoter, SV40 promoter, adenovirus promoter (major late promoter), pLλ promoter, CMV promoter, trp promoter, lac promoter, tac promoter, T7 promoter, vaccinia virus 7.5K promoter, tk promoter of HSV, and an animal cell promoter such as metallothionin promoter, beta-actin promoter, etc., but is not limited thereto.

The recombinant vector may comprise one or more selected from the group consisting of a polynucleotide encoding a polypeptide provided in the present disclosure, a gene encoding a target protein, and a gene encoding a secretion signal peptide.

The recombinant vector may comprise the polynucleotide, the gene encoding a target protein, and the gene encoding a secretion signal peptide.

Each of the polynucleotide, the gene encoding the target protein, and the gene encoding the secretion signal peptide may be operably linked to the promoter(s), and the promoter(s) may be the same as or different from each other.

The polypeptide provided in the present disclosure may have enhanced twin-arginine translocase activity. The polypeptide may have enhanced expression, secretion and/or production capability of a target protein.

In the present disclosure, the term "enhancement" of a polypeptide activity means that the activity of the polypeptide within a host cell (microorganism) is increased compared to an intrinsic activity. The enhancement may be used interchangeably with terms such as activation, up-regulation, overexpression, and increase. Here, activation, enhancement, up-regulation, overexpression, and increase may comprise both exhibition of an activity not originally present, and exhibition of an activity that is improved compared to the intrinsic activity or an activity before modification. The "intrinsic activity" refers to an activity of a specific polypeptide originally possessed by a parent strain or a non-modified microorganism before phenotypic change, when the phenotypic change is caused by a genetic mutation due to natural or artificial factors. This may be used interchangeably with "activity before modification". That an activity of a polypeptide is "enhanced", "up-regulated", "overexpressed", or "increased" compared to an intrinsic activity refers to an improvement compared to an activity and/or concentration of the specific polypeptide originally possessed by the parent strain or the non-modified microorganism before phenotypic change.

The enhancement may be achieved by introducing a heterologous (exogenous) polypeptide, or by enhancing the activity and/or concentration (expression level) of an endogenous polypeptide. Whether the activity of the polypeptide is enhanced may be confirmed from an increase in the activity level of the corresponding polypeptide, an increase in an expression level, or an increase in the secretion and/or production amount of a target protein linked to a secretion signal peptide recognized by the polypeptide.

Various methods well known in the art may be applied to the enhancement of the activity of the polypeptide, and the enhancement is not limited as long as it is capable of enhancing the activity of the target polypeptide compared to a microorganism before modification. Specifically, genetic engineering and/or protein engineering which are routine methods of molecular biology well known to those of ordinary skill in the art may be employed, but are not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be achieved by:
1) an increase in an intracellular copy number of a polynucleotide encoding the polypeptide;
2) a replacement of a gene expression regulatory region on a chromosome encoding the polypeptide with a sequence having strong activity;
3) a modification of a start codon of a gene transcript encoding the polypeptide or a nucleotide sequence encoding 5'-UTR region;
4) a modification of an amino acid sequence of the polypeptide to enhance the polypeptide activity;
5) a modification of a polynucleotide sequence encoding the polypeptide to enhance the polypeptide activity (e.g., modifying the polynucleotide sequence of the polypeptide gene to encode a polypeptide that is modified to enhance the activity);
6) an introduction of a heterologous polypeptide exhibiting the activity of the polypeptide or a heterologous polynucleotide encoding the same;
7) a codon optimization of a polynucleotide encoding the polypeptide;
8) selecting an exposed site by analyzing a tertiary structure of the polypeptide and modifying or chemically derivatizing it; or
9) a combination of one or two or more selected from 1) to 8), but is not particularly limited thereto.

More specifically,
the increase in the intracellular copy number of the polynucleotide encoding the polypeptide in the above item 1) may be achieved by introducing into a host cell a vector in which the polynucleotide encoding the polypeptide is operably linked and which is capable of replicating and functioning independently of the host. Alternatively, the increase may be achieved by introducing one copy or two or more copies of the polynucleotide encoding the corresponding polypeptide into the chromosome within the host cell. The introduction into the chromosome may be performed by introducing into the host cell a vector that is capable of inserting the polynucleotide into the chromosome of the host cell, but is not limited thereto. The vector is as described above.

The replacement of the gene expression regulatory region (or an expression regulatory sequence) on the chromosome encoding the polypeptide with the sequence having strong activity in the above item 2) may be, for example, an occurrence of a mutation in the sequence by deletion, insertion, non-conservative or conservative substitution, or a combination thereof so as to further enhance the activity of the expression regulatory region or a replacement with a sequence having stronger activity. The expression regulatory region may comprise, but is not particularly limited thereto, a promoter, an operator sequence, a sequence encoding a ribosome binding site, and a sequence regulating termination of transcription and translation. In one embodiment, the replacement may be replacement of the native promoter with a stronger promoter, but is not limited thereto.

Examples of a known strong promoter may be CJ1 to CJ7 promoters (US Patent No. US 7662943 B2), lac promoter, trp promoter, trc promoter, tac promoter, lambda phage PR promoter, PL promoter, tet promoter, gapA promoter, SPL7 promoter, SPL13(sm3) promoter (US Patent No. US 10584338 B2), O2 promoter (US Patent No. US 10273491 B2), tkt promoter, yccA promoter, etc., but are not limited thereto.

The modification of the start codon of the gene transcript encoding the polypeptide or the nucleotide sequence encoding 5'-UTR region in the above item 3) may be, for example, a substitution with a nucleotide sequence encoding another start codon having a higher polypeptide expression level compared to an endogenous start codon, but is not limited thereto.

The modification of the amino acid sequence or the polynucleotide sequence in the above items 4) and 5) may be an occurrence of a mutation on the sequence by deletion, insertion, non-conservative or conservative substitution, or a combination thereof in the amino acid sequence of the polypeptide or the polynucleotide sequence encoding the polypeptide so as to enhance the activity of the polypeptide, or a replacement with an amino acid sequence or a polynucleotide sequence improved so as to have stronger activity or an amino acid sequence or a polynucleotide sequence improved so as to have increased activity, but is not limited thereto. The replacement may be performed specifically by inserting the polynucleotide into a chromosome by homologous recombination, but is not limited thereto. The vector used in this case may further comprise a selection marker for confirming whether it is inserted into a chromosome. The selection marker is as described above.

The introduction of a heterologous polynucleotide exhibiting the activity of the polypeptide in the above item 6) may be the introduction of a heterologous polynucleotide encoding a polypeptide exhibiting the same or similar activity as the polypeptide into a host cell. The heterologous polynucleotide is not limited in origin or sequence as long as it exhibits the same or similar activity as the polypeptide. A method used for the introduction may be performed by those skilled in the art by appropriately selecting a known transformation method, and the polypeptide is produced by expression of the introduced polynucleotide in the host cell, thereby increasing the activity thereof.

The codon optimization of the polynucleotide encoding the polypeptide in the above item 7) may be codon optimization of an endogenous polynucleotide such that transcription or translation is increased within the host cell, or codon optimization of a heterologous polynucleotide such that optimized transcription or translation is performed within the host cell.

The modification or chemical derivatization by analyzing a tertiary structure of the polypeptide and selecting an exposed site in the above item 8) may be, for example, comparing sequence information of a polypeptide to be analyzed with sequence information of known proteins stored in a database to determine a template protein candidate based on a degree of sequence similarity and confirming a structure based thereon, and selecting an exposed site to be modified or chemically derivatized, and modifying or chemically derivatizing the same.

Such enhancement of the polypeptide activity may be an increase in the activity or concentration expression level of the corresponding polypeptide relative to the activity or concentration of the polypeptide expressed in a wild type or microorganism before modification, or an increase in the amount of a product produced from the corresponding polypeptide, but is not limited thereto.

### Microorganism

Still another aspect provides a microorganism comprising one or more selected from the group consisting of the polypeptide, the polynucleotide, and a vector comprising the polynucleotide.

In the present disclosure, the term "microorganism (or strain)" may comprise both a wild type microorganism and a microorganism that has undergone natural or artificial genetic modification. The microorganism may be a microorganism with a specific mechanism enhanced or attenuated due to reasons such as insertion of an exogenous gene or enhancement or attenuation of activity of an endogenous gene, and may be a microorganism comprising genetic modification for production of a target protein. In the present disclosure, the terms "microorganism", "strain", "host", and "host cell" may be used interchangeably.

The microorganism (or strain, recombinant cell) of the present disclosure may be a microorganism having twin-arginine translocase activity, or enhanced twin-arginine translocase activity, or expression, having secretion and/or production capability of a target protein, or having increased expression, secretion and/or production capability of a target protein.

As described above, in the microorganism, a target protein may be linked to a secretion signal peptide.

In the present disclosure, the term "non-modified microorganism" does not exclude a strain comprising a mutation that may occur naturally in a microorganism, and may refer to a wild type strain or natural strain itself, or a strain before a phenotypic change caused by genetic mutation due to natural or artificial factors. For example, the non-modified microorganism may refer to a strain into which a variant polypeptide of the present disclosure or a polynucleotide encoding the variant polypeptide has not been introduced, or a strain before the introduction. The "non-modified microorganism" may be used interchangeably with "pre-modification strain", "pre-modification microorganism", "non-variant strain", "non-modified strain", "non-variant microorganism" or "reference microorganism".

The microorganism may be a microorganism of the genus *Corynebacterium* (*Corynebacterium* sp.). The microorganism of the genus *Corynebacterium* may be one or more microorganisms selected from the group consisting of *Corynebacterium glutamicum*, *Corynebacterium crudilactis*, *Corynebacterium deserti*, *Corynebacterium efficiens*, *Corynebacterium callunae*, *Corynebacterium stationis*, *Corynebacterium singulare*, *Corynebacterium halotolerans*, *Corynebacterium striatum*, *Corynebacterium pollutisoli*, *Corynebacterium imitans*, *Corynebacterium testudinoris*, and *Corynebacterium flavescens,* but is not limited thereto.

In one embodiment, a control strain for comparing whether secretion and production capability of the target protein is increased may be a wild type microorganism of the genus *Corynebacterium* comprising a wild type twin-arginine translocase subunit TatC protein (e.g., the amino acid sequence of SEQ ID NO: 1), for example, a *Corynebacterium glutamicum* ATCC13032 strain.

In one embodiment, a microorganism having improved secretion and production capability of the target protein may exhibit an increase of 10% or more, 20% or more, 30% or more, 40% or more, 50% or more, 60% or more, 70% or more, 80% or more, 90% or more, 100% or more, 110% or more, 120% or more, 130% or more, 140% or more, 150% or more, 160% or more, 170% or more, 180% or more, 190% or more, 200% or more, 210% or more, 220% or more, 230% or more, 240% or more, 250% or more, 260% or more, 270% or more, 280% or more, 290% or more, or 300% or more in secretion and production capability of the target protein as compared with a parent strain before the mutation or a non-modified microorganism.

In one embodiment, the microorganism having improved secretion and production capability of the target protein may be about in secretion and production capability of the target protein as compared with the parent strain before the mutation or the non-modified microorganism.

The term "about" is a range comprising ±0.5, ±0.4, ±0.3, ±0.2, ±0.1, etc., and comprises all ranges of values that are equal to or similar to the numerical value following the term "about," but is not limited thereto.

### Composition for producing a target protein and production method

Still another aspect provides a composition for producing a target protein, comprising one or more selected from the group consisting of a microorganism and a medium in which the microorganism has been cultured.

Still another aspect provides a use of the microorganism in the production of a target protein.

The composition of the present disclosure may further comprise any appropriate excipient generally used in the composition for producing a target protein, and such an excipient may be, for example, a preservative agent, a wetting agent, a dispersing agent, a suspending agent, a buffering agent, a stabilizing agent, or an isotonic agent, but is not limited thereto.

Yet another aspect provides a method for producing (or preparing) a target protein, comprising culturing the microorganism in a medium.

The method for producing a target protein of the present disclosure may comprise culturing the microorganism in a medium.

In the present disclosure, the term "culturing" refers to growing the microorganism, for example, a *Corynebacterium glutamicum* strain, under appropriately controlled environmental conditions. The culturing process may be performed according to an appropriate medium and culture conditions known in the art. Such culturing process may be easily adjusted and used by those skilled in the art according to a selected strain. Specifically, the culturing may be performed in a batch manner, a continuous manner, and/or a fed-batch manner, but is not limited thereto.

In the present disclosure, the term "medium" refers to a material mixed with, as a main ingredient, nutrients required for culturing the microorganism, such as a *Corynebacterium glutamicum* strain, and supplies nutrients and growth factors, including water essential for survival and growth. Specifically, the medium and other culture conditions used for culturing the microorganism of the present disclosure may be used without any special limitation as long as they are a medium used for conventional culturing of a microorganism, but the microorganism of the present disclosure may be cultured in a conventional medium containing an appropriate carbon source, nitrogen source, phosphorus source, inorganic compound, amino acid and/or vitamin, etc. under an aerobic condition while controlling temperature, pH, etc.

Specifically, the culture medium for the microorganism, for example, a strain of the genus *Corynebacterium*, may be found in the literature ["Manual of Methods for General Bacteriology" by the American Society for Bacteriology (Washington D. Corynebacterium, USA, 1981)].

In the present disclosure, the carbon source may include carbohydrates such as glucose, saccharose, lactose, fructose, sucrose, maltose; sugar alcohols such as mannitol, sorbitol; organic acids such as pyruvic acid, lactic acid, citric acid; and amino acids such as glutamic acid, methionine, lysine. In addition, natural organic nutrients such as starch hydrolysate, molasses (e.g., blackstrap molasses), rice bran, cassava, sugarcane bagasse, and corn steep liquor may be used, specifically carbohydrates such as glucose and sterilized pretreated molasses (i.e., molasses converted to reducing sugar) may be used, and various carbon sources may be used without limitation in appropriate amounts. These carbon sources may be used alone or in a combination of two or more, but are not limited thereto.

Inorganic nitrogen sources such as ammonia, ammonium sulfate, ammonium chloride, ammonium acetate, ammonium phosphate, ammonium carbonate, ammonium nitrate; amino acids such as glutamic acid, methionine, glutamine; and organic nitrogen sources such as peptone, NZ-amine, meat extract, yeast extract, malt extract, corn steep liquor, casein hydrolysate, fish or decomposition products thereof, defatted soybean cake or decomposition products thereof may be used as the nitrogen source. These nitrogen sources may be used alone or in a combination of two or more, but are not limited thereto.

The phosphorus source may comprise monopotassium phosphate, dipotassium phosphate, or corresponding sodium-containing salts thereof, etc. Sodium chloride, calcium chloride, iron chloride, magnesium sulfate, iron sulfate, manganese sulfate, and calcium carbonate, etc. may be used as the inorganic compound, and amino acids, vitamins, and/or appropriate precursors may be included. These components or precursors may be added to a medium in a batch manner or continuous manner, but are not limited thereto.

In addition, during culturing of the microorganism, the pH of the medium may be adjusted by adding a compound such as ammonium hydroxide, potassium hydroxide, ammonia, phosphoric acid, and sulfuric acid to the medium in an appropriate manner. In addition, during culturing, an antifoam agent such as fatty acid polyglycol ester may be used to suppress foam generation. In addition, oxygen or oxygen-containing gas may be injected into the medium in order to maintain an aerobic state of the medium, or, in order to maintain an anaerobic or microaerobic state of the medium, the medium may be maintained without gas injection, or by injecting nitrogen, hydrogen, or carbon dioxide gas, but is not limited thereto.

In the culturing of the present disclosure, culturing temperature may be maintained at 20 to 45 °C, specifically 25 to 40 °C, and culturing may be performed for about 10 to 160 hours, but is not limited thereto.

A target protein produced by the culturing of the present disclosure may be secreted into the medium or may remain within cells.

The method for producing a target protein of the present disclosure may further comprise preparing the microorganism, preparing a medium for culturing the microorganism, or a combination thereof (in any order), for example, before the culturing step.

The method for producing a target protein of the present disclosure may further comprise recovering the target protein from the medium resulting from the culturing (medium in which culturing has been performed) or from the microorganism (e.g., a strain of the genus *Corynebacterium*). The recovery step may be further included after the culturing step.

The recovering may be collecting the desired target protein using an appropriate method known in the art according to the culturing method of the microorganism of the present disclosure, for example, batch, continuous, or fed-batch culturing methods. For example, centrifugation, filtration, treatment with a protein crystallizing precipitating agent (salting out method), extraction, ultrasonic disintegration, ultrafiltration, dialysis, various types of chromatography such as molecular sieve chromatography (gel filtration), adsorption chromatography, ion exchange chromatography, affinity chromatography, HPLC or a combination thereof may be used, and the desired target protein may be recovered from the medium or the microorganism using an appropriate method known in the art.

In addition, the method for producing a target protein of the present disclosure may further comprise a purification step. The purification may be performed using an appropriate method known in the art. In one embodiment, when the method for producing a target protein the present disclosure comprises both the recovery step and the purification step, the recovery step and the purification step may be performed continuously or non-continuously regardless of the order, or may be performed simultaneously or integrated into a single step, but are not limited thereto.

### [ADVANTAGEOUS EFFECTS]

The present disclosure provides a twin-arginine translocase subunit TatC variant, and a microorganism comprising the variant has excellent target protein secretion capability.

### [DESCRIPTION OF DRAWINGS]

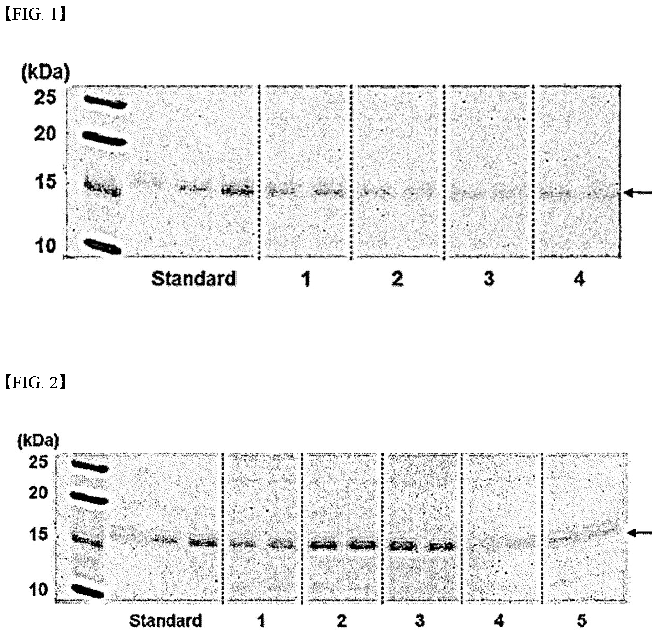

FIG. 1 shows the production concentration of a target protein (bCOL6_1) in a *Corynebacterium glutamicum* strain that expresses a wild type TatC protein or a TatC variant comprising a single amino acid substitution mutation (lane 1: wild type TatC protein; lane 2: TatC T106A variant; lane 3: TatC P107S variant; lane 4: TatC G108A variant).
FIG. 2 shows production concentration of a target protein (bCOL6_1) in a *Corynebacterium glutamicum* strain that expresses a wild type TatC protein or a TatC variant comprising a combined amino acid substitution mutation (lane 1: wild type TatC protein; lane 2: TatC T106A/P107S variant; lane 3: TatC P107S/G108A variant; lane 4: TatC T106A/G108A variant; lane 5: TatC T106A/P107S/G108A variant).

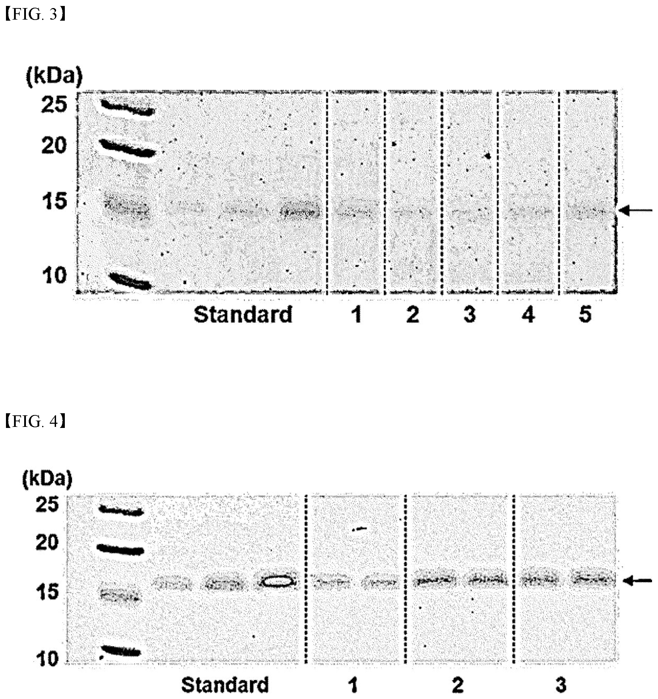

FIG. 3 shows production concentration of a target protein (bCOL6_1) in a *Corynebacterium* strain that expresses a *Corynebacterium glutamicum* TatC protein, a wild type TatC protein derived from *E*. *coli*, TatC P97S variant derived from *E*. *coli*, wild type TatC protein derived from Bacillus subtilis, TatC S95A/P96S/G97A variant derived from Bacillus subtilis (lane 1: *Corynebacterium glutamicum* TatC protein; lane 2: wild type TatC protein derived from *E. coli*; lane 3: TatC P97S variant derived from *E. coli*; lane 4: wild type TatC protein derived from Bacillus subtilis; lane 5: TatC S95A/P96S/G97A variant derived from Bacillus subtilis).
FIG. 4 shows the production concentration of the target protein (bCOL6_1) in a *Corynebacterium glutamicum* strain, in which a gene encoding TatC T106A/P107S variant or TatC T106A/P107S/G108A variant is introduced into the chromosome (lane 1: wild type; lane 2: TatC T106A/P107S variant; lane 3: TatC T106A/P107S/G108A variant).

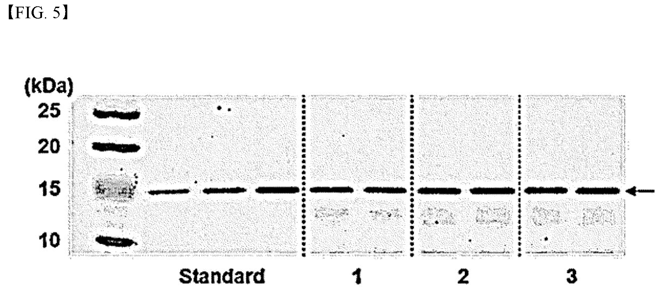

FIG. 5 shows the production concentration of the target protein (bCOL6_2) in a *Corynebacterium glutamicum* strain, in which a gene encoding TatC T106A/P107S variant or TatC T106A/P107S/G108A variant is introduced into the chromosome (lane 1: wild type; lane 2: TatC T106A/P107S variant; lane 3: TatC T106A/P107S/G108A variant).
FIG. 6 shows the production concentration of the target protein (YGK peptide) in a *Corynebacterium glutamicum* strain, in which a gene encoding TatC T106A/P107S variant or TatC T106A/P107S/G108A variant is introduced into the chromosome (lane 1: wild type; lane 2: TatC T106A/P107S variant; lane 3: TatC T106A/P107S/G108A variant).

### [EMBODIMENT OF INVENTION]

Hereinafter, the present invention will be described in more detail by the following Examples. However, these are only intended to exemplify the present invention, and the scope of the present invention is not limited by these Examples.

### Example 1. Production of the target protein using Corynebacterium glutamicum strain expressing TatC gene comprising a single amino acid mutation or a combined mutation

A twin-arginine translocase (Tat) system, which is a protein translocation system endogenous to a *Corynebacterium glutamicum* strain, serves to secrete a target protein having a secretion signal peptide sequence recognized by this system to the outside of a cell membrane. In order to increase production of the target protein using the *Corynebacterium glutamicum* strain, a strategy for improving secretion capability of the Tat system was explored. Among components of the Tat system, it is known that a TatC protein serves to proofread by recognizing a folding state of the target protein to be secreted and selecting and secreting only the protein having a correct folding state.

In the present disclosure, a TatC variant in which the amino acids of the 106th, the 107th, and/or the 108th residues of the *Corynebacterium glutamicum* TatC protein are substituted with different amino acids was prepared, and an effect of this on the target protein secretion capability of the *Corynebacterium glutamicum* strain expressing the the TatC variant was confirmed.

### Example 1-1. Establishment of co-expression vector of TatC gene comprising a single amino acid mutation and a model target protein

In order to compare the target protein secretion and production capability of the *Corynebacterium glutamicum* strain that expresses a *Corynebacterium glutamicum* TatC variant, an expression vector into which a partial sequence of collagen type 6 derived from bovine (bovine COL6A1) as a model target protein and *Corynebacterium glutamicum* TatC are co-introduced was prepared. Specifically, bovine COL6A1 sequence information [SEQ ID NO: 5] (Accession No. NP_001137337.1) was obtained from the National Institutes of Health (NIH) GenBank, and the C-terminus HisX6 tag (HHHHHH; SEQ ID NO: 75) for protein purification was added to some amino acid sequences among them, and named bCOL6_1 [SEQ ID NO: 6]. Based on the bCOL6_1 amino acid sequence, synthesis of a gene having the nucleotide sequence of [SEQ ID NO: 7] was performed after codon optimization. PCR was performed using the synthesized gene DNA as a template and the primers of [SEQ ID NO: 36] and [SEQ ID NO: 37] to obtain a bCOL6_1 gene fragment. PCR reaction was performed using Pfu-X DNA Polymerase (Solgent, Cat. No. SPX16-R500) according to the manufacturer's protocol.

Genomic DNA of a wild type *Corynebacterium glutamicum* ATCC13032 strain was extracted using a G-spin Total DNA extraction mini kit (Intron, Cat. No. 17045) according to a protocol provided in the kit, and PCR was performed using the genomic DNA as a template and the primers of [SEQ ID NO: 38] and [SEQ ID NO: 39] to obtain a TatC gene fragment. An amino acid sequence of a TatC protein of the wild type *Corynebacterium glutamicum* ATCC13032 strain is the amino acid sequence of SEQ ID NO: 1 (WP_011014400.1), and a gene sequence encoding the amino acid sequence is the nucleic acid sequence of SEQ ID NO: 2.

In order to secure the CgR0949 secretion signal peptide sequence [SEQ ID NO: 8] recognized by the Tat system, PCR was performed using ATCC13032 genomic DNA as a template and the primers of [SEQ ID NO: 40] and [SEQ ID NO: 41] to obtain a CgR0949 secretion signal peptide fragment. In order to secure a promoter sequence, PCR was performed using the CJ7 promoter (Pcj7) [SEQ ID NO: 10] (US Patent No. US 7662943 B2) as a template and the primers of [SEQ ID NO: 42] and [SEQ ID NO: 43] to obtain a Pcj7 fragment. In addition, in order to secure the pyk promoter (Ppyk) sequence [SEQ ID NO: 11], PCR was performed using the ATCC13032 genomic DNA as a template and the primers of [SEQ ID NO: 44] and [SEQ ID NO: 45] to obtain a Ppyk fragment.

The obtained Pcj7 fragment, the CgR0949 secretion signal peptide fragment, the bCOL6_1 gene fragment, the Ppyk fragment, the TatC gene fragment, and a pCES208 ("Construction of heat-inducible expression vector of Corynebacterium glutamicum and C. ammoniagenes: fusion of lambda operator with promoters isolated from C. ammoniagenes." Journal of microbiology and biotechnology 18.4 (2008): 639-647.) vector cleaved with XbaI restriction enzyme were cloned using In-fusion HD Cloning Kit (Takara Bio Inc, Cat. No. 638933) to obtain a recombinant vector, and were named "pCES208-Pcj7_CgR0949_bCOL6_1-Ppyk_tatC(WT)".

In order to obtain a TatC gene fragment comprising a single amino acid mutation, PCR was performed using the ATCC13032 genomic DNA as a template with the primers of [SEQ ID NO: 38] and [SEQ ID NO: 46], and the primers of [SEQ ID NO: 47] and [SEQ ID NO: 39], and gene fragments for a T106A variant in which threonine that is the 106th amino acid of the TatC protein is substituted with alanine were obtained. From the same template, PCR was performed using the primers of [SEQ ID NO: 38] and [SEQ ID NO: 48], and the primers of [SEQ ID NO: 49] and [SEQ ID NO: 39], and gene fragments for a P107S variant in which proline that is the 107th amino acid of the TatC protein is substituted with serine were obtained, and PCR was performed using the primers of [SEQ ID NO: 38] and [SEQ ID NO: 50], and the primers of [SEQ ID NO: 51] and [SEQ ID NO: 39], and gene fragments for a G108A variant in which glycine that is the 108th amino acid of the TatC protein is substituted with alanine were obtained. Each single amino acid variant gene fragment of the TatC and the Pcj7 fragment, the CgR0949 secretion signal peptide fragment, the bCOL6_1 gene fragment, the Ppyk fragment, and the pCES208 vector cleaved with XbaI restriction enzyme were cloned using In-fusion HD Cloning Kit to obtain recombinant vectors, and were named "pCES208-Pcj7_CgR0949_bCOL6_1-Ppyk_tatC(T106A)", "pCES208-Pcj7_CgR0949_bCOL6_1-Ppyk_tatC(P107S)", and "pCES208-Pcj7_CgR0949_bCOL6_1-Ppyk_tatC(G108A)", respectively.

The amino acid sequences of the TatC T106A variant, the TatC P107S variant, and the TatC G108A variant are the amino acid sequences of SEQ ID NO: 12, SEQ ID NO: 14, and SEQ ID NO: 16, respectively, and the gene sequences encoding the amino acid sequences are the nucleic acid sequences of SEQ ID NO: 13, SEQ ID NO: 15, and SEQ ID NO: 17, respectively.

### Example 1-2. Production of the target protein using Corynebacterium glutamicum strain expressing TatC gene comprising a single amino acid mutation

The recombinant vectors prepared in Example 1-1 were transformed into a *Corynebacterium glutamicum* wild type ATCC13032 strain through electroporation (Appl. Microbiol.Biotechnol. (1999) 52:541-545), and the strains into which the recombinant vectors were introduced were named "ATCC13032-bCOL6_1-tatC(WT)", "ATCC13032-bCOL6_1-tatC(T106A)", "ATCC13032-bCOL6_1-tatC(P107S)", and "ATCC13032-bCOL6_1-tatC(G108A)", respectively.

Culture evaluation was performed to confirm the target protein secretion and production capability of the four *Corynebacterium glutamicum* strains. Specifically, after subculturing a colony of each strain in a nutrient medium, each strain was inoculated into a 250 ml corner-baffle flask containing 25 ml of a production medium, and cultured with shaking at 30 °C for 48 hours at 200 rpm. Each medium comprised 50 mg/L of kanamycin antibiotic agent to maintain the recombinant vector.

### [Nutrient medium (pH 7.2)]

Glucose 10 g, meat extract 5 g, polypeptone 10 g, sodium chloride 2.5 g, yeast extract 5 g, agar 20 g, urea 2 g, kanamycin 50 mg (per 1 liter of distilled water)

### [Production medium (pH 7.0)]

Glucose 60g, ammonium sulfate 30g, yeast extract 10g, potassium phosphate dibasic 1g, magnesium sulfate heptahydrate 0.4g, iron sulfate heptahydrate 20mg, manganese sulfate pentahydrate 1.8mg, biotin 1.8mg, thiamine-HCl 9mg, calcium carbonate 50g, kanamycin 50mg (per 1 liter of distilled water)

After culturing was completed under the above conditions, production concentration of a bCOL6_1 target protein was confirmed through analysis of a culture supernatant. Specifically, a culture solution was centrifuged at 5000xg for 10 minutes to pellet the cells, and only the culture supernatant was separated and electrophoresis was performed on 4-20% Tris-Glycine SDS Precast Gel (Labis Koma, Cat. No. KG75355) using the SDS-PAGE (Laemmli, 1970) method. DIRECTBLUE^{™} gel staining solution (SCGBIOMAX, Cat. No. BDS-1000) was used to stain a gel, and an image of the stained gel was obtained using a Calibrated Densitometer (BIO-RAD, GS-900) and the concentration of the target protein in the culture solution was analyzed. In addition, it was confirmed by LC-MS analysis through the in gel digestion (Rosenfeld, 1992) method whether an amino acid sequence comprised in a protein band of a corresponding size was identical to the target protein. Production concentrations of the bCOL6_1 target protein of the four strains are shown in FIG. 1 and Table 1 below.

**[Table 1]**

| No. | Category | Strain name | Target protein concentration (mg/L) | Change amount compared to control group (%) |
|---|---|---|---|---|
| 1 | Control group | ATCC13032-bCOL6_1-tatC(WT) | 279.8 | - |
| 2 | Experimental group | ATCC13032-bCOL6_1- tatC(T106A) | 165.7 | -40.78% |
| 3 | | ATCC13032-bCOL6_1- tatC(P107S) | 202.2 | -27.73% |
| 4 | | ATCC13032-bCOL6_1- tatC(G108A) | 228.9 | -18.19% |

As shown in Table 1, a strain that expresses the TatC variant comprising a single amino acid substitution mutation did not exhibit an increase in the concentration of the target protein (bCOL6_1) compared to a strain that expresses the wild type TatC protein (ATCC13032-bCOL6_1-tatC(WT)), which is a control group.

### Example 1-3. Production of the target protein using Corynebacterium glutamicum strain expressing TatC gene comprising combined amino acid mutations

Recombinant vectors were established for evaluating an effect on the target protein secretion and production capability of the *Corynebacterium glutamicum* strain when combining the single amino acid substitution mutations of Example 1-1.

Specifically, PCR was performed using the genomic DNA of the *Corynebacterium glutamicum* ATCC13032 strain as a template with the primers of [SEQ ID NO: 38] and [SEQ ID NO: 52], and the primers of [SEQ ID NO: 53] and [SEQ ID NO: 39], and gene fragments for a T106A, P107S combined variant were obtained. From the same template, PCR was performed using the primers of [SEQ ID NO: 38] and [SEQ ID NO: 54], and the primers of [SEQ ID NO: 55] and [SEQ ID NO: 39], and gene fragments for a P107S, G108A combined variant were obtained, and PCR was performed using the primers of [SEQ ID NO: 38] and [SEQ ID NO: 56], and the primers of [SEQ ID NO: 57] and [SEQ ID NO: 39], and gene fragments for a T106A, G108A combined variant were obtained. Likewise, from the same template, PCR was performed using the primers of [SEQ ID NO: 38] and [SEQ ID NO: 58], and the primers of [SEQ ID NO: 59] and [SEQ ID NO: 39], and gene fragments for a T106A, P107S, and G108A combined variant were obtained.

The amino acid combined variant gene fragments of the TatC; the Pcj7 fragment, the CgR0949 secretion signal peptide fragment, the bCOL6_1 gene fragment, and the Ppyk fragment obtained in Example 1-1; and the pCES208 vector cleaved with XbaI restriction enzyme were cloned using In-fusion HD Cloning Kit to obtain recombinant vectors. The recombinant vectors were named "pCES208-Pcj7_CgR0949_bCOL6_1-Ppyk_tatC(T106A/P107S)", "pCES208-Pcj7_CgR0949_bCOL6_1-Ppyk_tatC(P107S/G108A)", "pCES208-Pcj7_CgR0949_bCOL6_1-Ppyk_tatC(T106A/G108A)", and "pCES208-Pcj7_CgR0949_bCOL6_1-Ppyk_tatC(T106A/P107S/G108A)", respectively.

The amino acid sequences of the TatC T106A/P107S variant, the TatC P107S/G108A variant, the TatC T106A/G108A variant, and the TatC T106A/P107S/G108A variant are the amino acid sequences of SEQ ID NO: 18, SEQ ID NO: 20, SEQ ID NO: 22, and SEQ ID NO: 24, respectively, and the gene sequences encoding the amino acid sequences are the nucleic acid sequences of SEQ ID NO: 19, SEQ ID NO: 21, SEQ ID NO: 24, and SEQ ID NO: 25, respectively.

The prepared recombinant vectors were transformed into the wild type *Corynebacterium glutamicum* ATCC13032 strain through electroporation, and the strains into which the recombinant vectors were introduced were named "ATCC13032-bCOL6_1-tatC(T106A/P107S)", "ATCC13032-bCOL6_1-tatC(P107S/G108A)", "ATCC13032-bCOL6_1-tatC(T106A/G108A)", and "ATCC13032-bCOL6_1-tatC(T106A/P107S/G108A)", respectively. In order to compare the target protein secretion and production capability of the four *Corynebacterium glutamicum* strains and the ATCC13032-bCOL6_1-tatC(WT) strain secured in Example 1-2, culturing was performed in the same manner as in Example 1-2, and the concentrations of the target protein in the culture solution were analyzed, which are shown in FIG. 2 and Table 2 below.

**[Table 2]**

| No. | Category | Strain name | Target protein concentration (mg/L) | Change amount compared to control group (%) |
|---|---|---|---|---|
| 1 | Control group | ATCC13032-bCOL6_1-tatC(WT) | 278.6 | - |
| 2 | Experimental group | ATCC13032-bCOL6_1- tatC(T106A/P107S/G108A) | 771.3 | 176.85% |
| 3 | | ATCC13032-bCOL6_1-tatC(T106A/P107S) | 781.2 | 180.4% |
| 4 | | ATCC13032-bCOL6_1-tatC(P107S/G108A) | 201 | -27.85% |
| 5 | | ATCC13032-bCOL6_1-tatC(T106A/G108A) | 267.2 | -4.09% |

As shown in Table 2, among the strains that express the TatC variant comprising amino acid combined substitution mutations, an ATCC13032-bCOL6_1-tatC(T106A/P107S) strain and an ATCC13032-bCOL6_1-tatC(T106A/P107S/G108A) strain exhibited higher target protein concentration of about 2.8-fold compared to the strain that expresses the wild type TatC protein (ATCC13032-bCOL6_1-tatC(WT)), which is a control group.

### Example 2. Production of target protein using Corynebacterium glutamicum strain expressing TatC gene derived from heterologous microorganism comprising combined amino acid mutations

A result of Example 1 showed that the T106A/P107S variant and the T106A/P107S/G108A variant of the *Corynebacterium glutamicum* TatC improved the target protein secretion and production capability of the *Corynebacterium glutamicum* strain. It was confirmed whether the same target protein secretion and production capability improvement effect was exhibited when an equivalent mutation was applied to a TatC gene derived from a heterologous microorganism as an expansion therefrom. Specifically, amino acid sequence information [SEQ ID NO: 3; WP_000109943.1] encoded by the TatC gene [SEQ ID NO: 4] of *E*. *coli*, and amino acid sequence information [SEQ ID NO: 26; WP_010886426.1] encoded by the TatC gene [SEQ ID NO: 27] of Bacillus subtilis were obtained from the National Institutes of Health GenBank, and BLAST of the National Institutes of Health was used for homology analysis with amino acid sequence information [SEQ ID NO: 1; WP_011014400.1] encoded by the TatC gene [SEQ ID NO: 2] of the *Corynebacterium glutamicum*. From the results, it was confirmed that proline that is the 97th amino acid of the TatC protein derived from *E*. *coli*, and proline that is the 96th amino acid of the TatC protein derived from Bacillus subtilis correspond to proline that is the 107th amino acid of the TatC derived from the *Corynebacterium glutamicum*. Since the 96th amino acid and the 98th amino acid of the TatC protein derived from *E. coli* are alanine, a TatC P97S variant in which proline that is the 97th amino acid is singly substituted with serine was prepared, and an S95A/P96S/G97A variant in which serine that is the 95th amino acid of the TatC protein derived from Bacillus subtilis is substituted with alanine, proline that is the 96th amino acid is substituted with serine, and glycine that is the 97th amino acid is substituted with alanine was prepared. Specifically, genomic DNA of an *E. coli* K-12 KCTC 2223 strain and a Bacillus subtilis KCTC 3135 strain was extracted using a G-spin Total DNA extraction mini kit according to a protocol provided in the kit. PCR was performed using the genomic DNA of the extracted *E. coli* K-12 KCTC 2223 strain as a template with the primers of [SEQ ID NO: 60] and [SEQ ID NO: 61] to obtain a wild type *E. coli* TatC gene fragment, and from the same template, PCR was performed using the primers of [SEQ ID NO: 60] and [SEQ ID NO: 62], and the primers of [SEQ ID NO: 63] and [SEQ ID NO: 61], and gene fragments for an *E. coli* TatC P97S variant were obtained. Likewise, PCR was performed using the genomic DNA of the extracted Bacillus subtilis KCTC 3135 strain as a template, with the primers of [SEQ ID NO: 64] and [SEQ ID NO: 65] to obtain a wild type Bacillus subtilis TatC gene fragment, and from the same template, PCR was performed using the primers of [SEQ ID NO: 64] and [SEQ ID NO: 66], and the primers of [SEQ ID NO: 67] and [SEQ ID NO: 65], and gene fragments for a Bacillus subtilis TatC S95A/P96S/G97A variant were obtained.

The wild type gene fragment and P97S variant gene fragments of the *E. coli* TatC, and the wild type gene fragment and S95A/P96S/G97A variant gene fragments of the Bacillus subtilis TatC; the Pcj7 fragment, the CgR0949 secretion signal peptide fragment, the bCOL6_1 gene fragment, and the Ppyk fragment obtained in Example 1-1; and the pCES208 vector cleaved with XbaI restriction enzyme were cloned using In-fusion HD Cloning Kit to obtain recombinant vectors. The recombinant vectors were named "pCES208-Pcj7_CgR0949_bCOL6_1-Ppyk_EctatC(WT)", "pCES208-Pcj7_CgR0949_bCOL6_1-Ppyk_EctatC(P97S)", "pCES208-Pcj7_CgR0949_bCOL6_1-Ppyk_BstatC(WT)", and "pCES208-Pcj7_CgR0949_bCOL6_1-Ppyk_BstatC(S95A/P96S/G97A)", respectively.

The amino acid sequences of the TatC P97S variant derived from *E. coli* and the TatC S95A/P96S/G97A variant derived from Bacillus subtilis are the amino acid sequences of SEQ ID NO: 28 and SEQ ID NO: 30, respectively, and the gene sequences encoding the amino acid sequences are the nucleic acid sequences of SEQ ID NO: 29 and SEQ ID NO: 31, respectively.

The prepared recombinant vectors were transformed into the *Corynebacterium glutamicum* wild type ATCC13032 strain through electroporation, and the strains into which the recombinant vectors were introduced were named "ATCC13032-bCOL6_1-EctatC(WT)", "ATCC13032-bCOL6_1-EctatC(P97S)", "ATCC13032-bCOL6_1-BstatC(WT)", and "ATCC13032-bCOL6_1-BstatC(S95A/P96S/G97A)", respectively. In order to compare the target protein secretion and production capability of the four *Corynebacterium glutamicum* strains and the ATCC13032-bCOL6_1-tatC(WT) strain secured in Example 1-2, culturing was performed in the same manner as in Example 1-2, and the concentrations of the target protein in the culture solution were analyzed, which are shown in FIG. 3 and Table 3 below.

**[Table 3]**

| No. | Category | Strain name | Target protein concentration (mg/L) | Change amount compared to control group (%) |
|---|---|---|---|---|
| 1 | Control group | ATCC13032-bCOL6_1-tatC(WT) | 266.3 | - |
| 2 | Experimental group | ATCC13032-bCOL6_1-EctatC(WT) | 116.1 | -56.40% |
| 3 | | ATCC13032-bCOL6_1-EctatC(P97S) | 116.5 | -56.25% |
| 4 | | ATCC13032-bCOL6_1-BstatC(WT) | 164.5 | -38.23% |
| 5 | | ATCC13032-bCOL6_1- BstatC(S95A/P96S/G97A) | 179.3 | -32.67% |

As shown in Table 3, it was confirmed that the *Corynebacterium glutamicum* strain (ATCC13032-bCOL6_1-EctatC(WT)) that expresses the *E. coli* TatC or the *Corynebacterium glutamicum* strain (ATCC13032-bCOL6_1-BstatC(WT)) that expresses the Bacillus subtilis TatC exhibited a lower target protein concentration than the strain that expresses the *Corynebacterium glutamicum* TatC (ATCC13032-bCOL6_1-tatC(WT)) as a control. In addition, the *Corynebacterium glutamicum* strain (ATCC13032-bCOL6_1-EctatC (P97S)) into which the *E. coli* TatC P97S variant corresponding to the P107S mutation of the *Corynebacterium glutamicum* TatC was introduced, and the *Corynebacterium glutamicum* strain (ATCC13032-bCOL6_1-BstatC(S95A/P96S/G97A)) into which the Bacillus subtilis TatC S95A/P96S/G97A variant corresponding to the T106A/9107S/G108A mutation of the *Corynebacterium glutamicum* TatC was introduced, did not exhibit a significant increase in target protein concentration compared to the *Corynebacterium glutamicum* strain expressing the wild type *E. coli* TatC and the *Corynebacterium glutamicum* strain expressing the wild type Bacillus subtilis TatC, respectively.

These results indicate that the target protein secretion and production capability improvement effect of the *Corynebacterium glutamicum* TatC variants confirmed in Example 1-3 is not universally exhibited for TatC derived from a heterologous microorganism.

### Example 3. Preparation of a modified Corynebacterium glutamicum strain in which a TatC gene comprising combined amino acid mutations is introduced into the chromosome, and production of various target proteins using the same

A modified strain was prepared by introducing the *Corynebacterium glutamicum* TatC T106A/P107S variant or the TatC T106A/P107S/G108A variant into the chromosome, which was confirmed in Example 1 to improve the target protein secretion and production capability of the *Corynebacterium glutamicum* strain. Specifically, PCR was performed using the pCES208-Pcj7_CgR0949_bCOL6_1-Ppyk_tatC(T106A/P107S) or pCES208-Pcj7_CgR0949_bCOL6_1-Ppyk_tatC(T106A/P107S/G108A) vector DNA as a template and the primers of [SEQ ID NO: 68] and [SEQ ID NO: 69], and gene fragments for the TatC T106A/P107S variant and the TatC T106A/P107S/G108A variant were obtained, respectively. The recombinant vectors were obtained by cloning the TatC combined mutant gene fragment and a pDZ vector (US Patent No. US 9109242 B2) cleaved with XbaI restriction enzyme using In-fusion HD Cloning Kit, and were introduced into chromosomes through homologous recombination by transforming into the wild type *Corynebacterium glutamicum* ATCC13032 strain through electroporation, respectively. Thereafter, PCR was performed on colonies that went through a secondary crossover process using the primers of [SEQ ID NO: 68] and [SEQ ID NO: 69] to confirm the improved strains in which the wild type TatC gene on the chromosome is substituted with the combined-mutant gene, and were named "ATCC13032::tatC(T106A/P107S)" and "ATCC13032::tatC(T106A/P107S/G108A)", respectively.

In order to compare the target protein secretion and production capability of the *Corynebacterium glutamicum* strain into which the TatC variant was introduced, an expression vector of the bCOL6_1 target protein was prepared. Specifically, PCR was performed using the pCES208-Pcj7_CgR0949_bCOL6_1-Ppyk_tatC(WT) vector DNA as a template and the primers of [SEQ ID NO: 42] and [SEQ ID NO: 70], and a gene fragment for a Pcj7_CgR0949_bCOL6_1 expression cassette was obtained. The recombinant vector was obtained by cloning the gene fragment and the pCES208 vector cleaved with XbaI restriction enzyme using In-fusion HD Cloning Kit, and was named "pCES208-Pcj7_CgR0949_bCOL6_1". The pCES208-Pcj7_CgR0949_bCOL6_1 recombinant vector was transformed into the *Corynebacterium glutamicum* wild type ATCC13032 strain and the ATCC13032::tatC(T106A/P107S), ATCC13032::tatC(T106A/P107S/G108A) modified strains through electroporation, and the strains into which the recombinant vector was introduced were named "ATCC13032-bCOL6_1", "ATCC13032::tatC(T106A/P107S)-bCOL6_1", and "ATCC13032::tatC(T106A/P107S/G108A)-bCOL6_1", respectively. In order to compare the target protein secretion and production capability of the three *Corynebacterium glutamicum* strains, culturing was performed in the same manner as in Example 1-2, and the concentrations of the target protein in the culture solution were analyzed, which are shown in FIG. 4 and Table 4 below.

**[Table 4]**

| No. | Category | Strain name | Target protein concentration (mg/L) | Change amount compared to control group (%) |
|---|---|---|---|---|
| 1 | Control group | ATCC13032-bCOL6_1 | 85.3 | - |
| 2 | Experimental group | ATCC13032::tatC(T106A/P107S)-bCOL6_1 | 193.1 | 126.38% |
| 3 | | ATCC13032::tatC(T106A/P107S/G108A)-bCOL6_1 | 188.4 | 120.87% |

As shown in Table 4, it was confirmed that the *Corynebacterium glutamicum* strain (ATCC13032::tatC(T106A/P107S)-bCOL6_1) in which the *Corynebacterium glutamicum* TatC T106A/P107S variant was introduced into the chromosome, and the *Corynebacterium glutamicum* strain (ATCC13032::tatC(T106A/P107S/G108A)-bCOL6_1) in which the *Corynebacterium glutamicum* TatC T106A/P107S/G108A variant was introduced into the chromosome exhibited higher target protein concentration of two-fold or higher compared to the control group (ATCC13032-bCOL6_1).

From this result, it was confirmed that the *Corynebacterium glutamicum* strain in which the TatC T106A/P107S variant or the T106A/P107S/G108A variant was introduced into the chromosome to express the same exhibited improved target protein secretion and production capability.

In order to confirm whether the target protein secretion and production capability improvement effect exhibited by a *Corynebacterium glutamicum* strain in which a TatC variant was introduced into the chromosome is likewise exhibited for target proteins other than bCOL6_1, expression vectors for target proteins other than bCOL6_1 were prepared. Specifically, among the bovine COL6A1 sequence information, the C-terminal HisX6 tag (HHHHHH; SEQ ID NO: 75) for protein purification was added to some amino acid sequences different from bCOL6_1, and the resulting sequence was named bCOL6_2 [SEQ ID NO: 32]. After codon optimization, a gene having the nucleic acid sequence of [SEQ ID NO: 33] was synthesized. PCR was performed using the synthesized DNA as a template with the primers of [SEQ ID NO: 71] and [SEQ ID NO: 72], and a bCOL6_2 gene fragment was obtained. Meanwhile, in order to prepare an expression vector of a YGK peptide (comprising C-terminal HisX6 tag) [SEQ ID NO: 34] [Mirzaei et al., Journal of Functional Foods, 2015, Journal of Functional Foods Volume 19, Part A, December 2015, Pages 259-268], which is an antioxidant-functional protein derived from *Saccharomyces cerevisiae*, a gene having the nucleic acid sequence of [SEQ ID NO: 35] was synthesized after codon optimization, likewise, and PCR was performed using the synthesized DNA as a template with the primers of [SEQ ID NO: 73] and [SEQ ID NO: 74] to obtain a YGK gene fragment. Each of the gene fragments; the Pcj7 fragment and the CgR0949 secretion signal peptide fragment obtained in Example 1-1; and the pCES208 vector cleaved with XbaI restriction enzyme were cloned using In-fusion HD Cloning Kit to obtain recombinant vectors. The recombinant vectors were named "pCES208-Pcj7_CgR0949_bCOL6_2" and "pCES208-Pcj7_CgR0949_YGK", respectively.

The recombinant vectors were transformed into the wild type *Corynebacterium glutamicum* ATCC13032 strain and the ATCC13032::tatC(T106A/P107S), ATCC13032::tatC(T106A/P107S/G108A) modified strains through electroporation, and the strains into which the recombinant vectors were introduced were named "ATCC13032-bCOL6_2", "ATCC13032::tatC(T106A/P107S)-bCOL6_2", "ATCC13032::tatC(T106A/P107S/G108A)-bCOL6_2", "ATCC13032-YGK", "ATCC13032::tatC(T106A/P107S)-YGK", and "ATCC13032::tatC(T106A/P107S/G108A)-YGK", respectively.

First, in order to compare the target protein secretion and production capability of the three *Corynebacterium glutamicum* strains into which the bCOL6_2 expression vector was introduced, culturing was performed in the same manner as in Example 1-2, and the concentrations of the target protein in the culture solution were analyzed, which are shown in FIG. 5 and Table 5 below.

**[Table 5]**

| No. | Category | Strain name | Target protein concentration (mg/L) | Change amount compared to control group (%) |
|---|---|---|---|---|
| 1 | Control group | ATCC13032-bCOL6_2 | 110.6 | - |
| 2 | Experimental group | ATCC13032::tatC(T106A/P107S)-bCOL6_2 | 176.5 | 59.59% |
| 3 | | ATCC13032::tatC(T106A/P107S/G108A)-bCOL6_2 | 165.8 | 49.91% |

As shown in Table 5, it was confirmed that the *Corynebacterium glutamicum* strain (ATCC13032::tatC(T106A/P107S)-bCOL6_2) in which the TatC T106A/P107S variant was introduced into the chromosome, and the *Corynebacterium glutamicum* strain (ATCC13032::tatC(T106A/P107S/G108A)-bCOL6_2) in which the TatC T106A/P107S/ G108A variant was introduced into the chromosome exhibited higher target protein concentration of 1.5-fold or higher for the bCOL6_2 target protein compared to the control strain (ATCC13032-bCOL6_2).

Next, in order to compare the target protein secretion and production capability of the three *Corynebacterium glutamicum* strains into which the YGK expression vector was introduced, culturing was performed in the same manner as in Example 1-2, and the concentrations of the target protein in the culture solution were analyzed, which are shown in FIG. 6 and Table 6 below.

**[Table 6]**

| No. | Category | Strain name | Target protein concentration (mg/L) | Change amount compared to control group (%) |
|---|---|---|---|---|
| 1 | Control group | ATCC13032-YGK | 49.4 | - |
| 2 | Experimental group | ATCC13032::tatC(T106A/P107S)-YGK | 134.0 | 171.26% |
| 3 | | ATCC13032::tatC(T106A/P107S/G108A)-YGK | 148.6 | 200.81% |

As shown in Table 6, it was confirmed that the *Corynebacterium glutamicum* strain (ATCC13032::tatC(T106A/P107S)-bCOL6_2) in which the TatC T106A/P107S variant was introduced into the chromosome, and the *Corynebacterium glutamicum* strain (ATCC13032::tatC(T106A/P107S/G108A)-bCOL6_2) in which the TatC T106A/P107S/G108A variant was introduced into the chromosome exhibited higher target protein concentration of 2.7-fold and 3-fold, respectively, for the YGK target protein compared to the control group (ATCC13032-bCOL6_2).

From the result, it was confirmed that the *Corynebacterium glutamicum* strain in which the TatC T106A/P107S variant or the TatC T106A/P107S/G108A variant was introduced into the chromosome exhibited improved target protein secretion and production capability for target proteins of various sequences and types.

From the above description, those skilled in the art to which the present invention pertains will be able to understand that the present invention can be embodied into different and more detailed modes, without departing from the technical spirit or essential features thereof. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A polypeptide comprising an amino acid sequence having 90% or more sequence homology to the amino acid sequence of SEQ ID NO: 1,
wherein (1) amino acids corresponding to the 106th and the 107th residues from the N-terminus in the amino acid sequence of SEQ ID NO: 1 are substituted with different amino acids, or
(2) amino acids corresponding to the 106th, the 107th, and the 108th residues from the N-terminus in the amino acid sequence of SEQ ID NO: 1 are substituted with different amino acids.

2. The polypeptide according to claim 1, wherein
(1) the amino acid corresponding to the 106th residue from the N-terminus in the amino acid sequence of SEQ ID NO: 1 is substituted with alanine, arginine, histidine, lysine, aspartic acid, glutamic acid, serine, asparagine, glutamine, cysteine, glycine, proline, valine, isoleucine, leucine, methionine, phenylalanine, tyrosine, or tryptophan; and
the amino acid corresponding to the 107th residue is substituted with serine, arginine, histidine, lysine, aspartic acid, glutamic acid, threonine, asparagine, glutamine, cysteine, glycine, alanine, valine, isoleucine, leucine, methionine, phenylalanine, tyrosine, or tryptophan; or
(2) the amino acid corresponding to the 106th residue from the N-terminus in the amino acid sequence of SEQ ID NO: 1 is substituted with alanine, arginine, histidine, lysine, aspartic acid, glutamic acid, serine, asparagine, glutamine, cysteine, glycine, proline, valine, isoleucine, leucine, methionine, phenylalanine, tyrosine, or tryptophan;
the amino acid corresponding to the 107th residue is substituted with serine, arginine, histidine, lysine, aspartic acid, glutamic acid, threonine, asparagine, glutamine, cysteine, glycine, alanine, valine, isoleucine, leucine, methionine, phenylalanine, tyrosine, or tryptophan; and
the amino acid corresponding to the 108th residue is substituted with alanine, arginine, histidine, lysine, aspartic acid, glutamic acid, serine, threonine, asparagine, glutamine, cysteine, proline, valine, isoleucine, leucine, methionine, phenylalanine, tyrosine, or tryptophan.

3. The polypeptide according to claim 1, wherein
(1) the amino acid corresponding to the 106th residue from the N-terminus in the amino acid sequence of SEQ ID NO: 1 is substituted with alanine and the amino acid corresponding to the 107th residue is substituted with serine; or
(2) the amino acid corresponding to the 106th residue from the N-terminus in the amino acid sequence of SEQ ID NO: 1 is substituted with alanine, the amino acid corresponding to the 107th residue is substituted with serine, and the amino acid corresponding to the 108th residue is substituted with alanine.

4. The polypeptide according to claim 1, wherein the polypeptide has twin-arginine translocase activity.

5. The polypeptide according to claim 1, wherein the polypeptide is a twin-arginine translocase subunit TatC protein.

6. The polypeptide according to claim 1, wherein the polypeptide is derived from a microorganism of the genus *Corynebacterium*.

7. The polypeptide according to claim 6, wherein the microorganism of the genus *Corynebacterium* is *Corynebacterium glutamicum*.

8. The polypeptide according to claim 1, comprising the amino acid sequence of SEQ ID NO: 1, wherein (1) the 106th and the 107th residues in the amino acid sequence of SEQ ID NO: 1 are substituted with different amino acids; or
(2) the 106th, the 107th, and the 108th residues in the amino acid sequence of SEQ ID NO: 1 are substituted with different amino acids.

9. The polypeptide according to claim 8, wherein
(1) the 106th residue from the N-terminus in the amino acid sequence of SEQ ID NO: 1 is substituted with alanine, arginine, histidine, lysine, aspartic acid, glutamic acid, serine, asparagine, glutamine, cysteine, glycine, proline, valine, isoleucine, leucine, methionine, phenylalanine, tyrosine, or tryptophan; and
the 107th residue is substituted with serine, arginine, histidine, lysine, aspartic acid, glutamic acid, threonine, asparagine, glutamine, cysteine, glycine, alanine, valine, isoleucine, leucine, methionine, phenylalanine, tyrosine, or tryptophan; or
(2) the 106th residue from the N-terminus in the amino acid sequence of SEQ ID NO: 1 is substituted with alanine, arginine, histidine, lysine, aspartic acid, glutamic acid, serine, asparagine, glutamine, cysteine, glycine, proline, valine, isoleucine, leucine, methionine, phenylalanine, tyrosine, or tryptophan;
the 107th residue is substituted with serine, arginine, histidine, lysine, aspartic acid, glutamic acid, threonine, asparagine, glutamine, cysteine, glycine, alanine, valine, isoleucine, leucine, methionine, phenylalanine, tyrosine, or tryptophan; and
the 108th residue is substituted with alanine, arginine, histidine, lysine, aspartic acid, glutamic acid, serine, threonine, asparagine, glutamine, cysteine, proline, valine, isoleucine, leucine, methionine, phenylalanine, tyrosine, or tryptophan.

10. The polypeptide according to claim 8, wherein
(1) the 106th residue from the N-terminus in the amino acid sequence of SEQ ID NO: 1 is substituted with alanine and the 107th residue is substituted with serine; or
(2) the 106th residue from the N-terminus in the amino acid sequence of SEQ ID NO: 1 is substituted with alanine, the 107th residue is substituted with serine, and the 108th residue is substituted with alanine.

11. The polypeptide according to claim 8, comprising the amino acid sequence of SEQ ID NO: 18 or SEQ ID NO: 24.

12. A polynucleotide encoding a polypeptide according to any one of claims 1 to 11.

13. A recombinant vector comprising the polynucleotide according to claim 12.

14. The recombinant vector according to claim 13, further comprising a gene encoding a target protein.

15. The recombinant vector according to claim 14, wherein the target protein is one or more selected from the group consisting of collagen, polypeptide derived from collagen, hormone, cytokine, antibody, antibiotic peptide, and antioxidant peptide.

16. A microorganism for producing a target protein, comprising:
the polypeptide according to claim 1 and the target protein; or
a polynucleotide encoding the polypeptide and a gene encoding the target protein.

17. The microorganism according to claim 16, wherein the microorganism has enhanced secretion and production capability of the target protein.

18. The microorganism according to claim 16, wherein the microorganism is a microorganism of the genus *Corynebacterium*.

19. The microorganism according to claim 18, wherein the microorganism of the genus *Corynebacterium* is *Corynebacterium glutamicum*.

20. A composition for producing a target protein, comprising one or more selected from the group consisting of the microorganism according to any one of claims 16 to 19 and a medium in which the microorganism is cultured.

21. A method for producing a target protein, comprising culturing the microorganism according to any one of claims 16 to 19 in a medium.

22. The method according to claim 21, further comprising, after the culturing step, recovering the target protein from the cultured microorganism, the medium, or both.
